# FASCICULE DE BREVET EUROPEEN

(11) **EP 0 976 390 B1**
(45) Date de publication et mention de la délivrance du brevet: **14.11.2001**
(21) Numéro de dépôt: 99401624.4
(22) Date de dépôt: 30.06.1999
(51) Int. Cl.: A61K 7/48

(54) **Composition cosmétique solide et ses utilisations**
Feste kosmetische Zubereitung und ihre Anwendungen
Solid cosmetic composition and its uses

(30) Priorité: 30.07.1998 FR 9809793
(43) Date de publication de la demande: 02.02.2000
(73) Titulaire: L'OREAL, 75008 Paris (FR)
(72) Inventeur: Roulier, Véronique, 75010 Paris (FR); Quemin, Eric, 93290 Tremblay en France (FR)
(74) Mandataire: Rasson, Catherine

(56) Documents cités:
- EP-A- 0 667 148
- EP-A- 0 803 245
- EP-A- 0 930 017
- WO-A-97/17053
- WO-A-97/17055
- GB-A- 2 219 803
- US-A- 4 647 470
- US-A- 4 746 528
- US-A- 5 342 626
- H-D. BELITZ, W. GROSCH: 'Lehrbuch der Lebensmittelchemie', 1992, SPRINGER-VERLAG, DEUTSCHLAND Vierte, überarbeitete Auflage * page 499 - page 502 *

## Description

La présente invention concerne une composition solide pour application topique, ainsi que son utilisation dans les domaines cosmétique et/ou dermatologique, notamment pour le soin et/ou le traitement de la peau, du cuir chevelu, des cheveux et/ou des muqueuses, pour le maquillage de la peau et/ou des fibres kératiniques telles que les cils et les cheveux, pour le coiffage et/ou la mise en forme des fibres kératiniques, et en particulier des cheveux.

On connaît dans l'industrie cosmétique des produits se présentant sous forme solide. Comme produits de ce type, on peut citer par exemple dans le domaine du maquillage, les bâtons ou "sticks" de rouge à lèvres, de fond de teint ou d'ombre à paupières ; dans le domaine du soin de la peau ou des lèvres, les crayons réparateurs des lèvres, les bâtons ou "sticks" dépigmentants, démaquillants ou hydratants ; dans le domaine de l'hygiène, les sticks déodorants, les sticks ou les pains moussants pour le rasage ou pour le lavage de la peau.

Comme produits solides, on peut aussi citer les patchs qui agissent notamment par transdermie par exemple soit pour faire pénétrer un actif dans la peau soit pour nettoyer la peau.

Les sticks formulés à base de cires présentent certains inconvénients : ils ont un caractère gras qui n'est pas apprécié par les utilisateurs et ils manquent de fraîcheur à l'application. En outre, il est difficile d'y introduire des actifs hydrophiles.

Par ailleurs, les sticks non gras tels que les sticks déodorants contiennent généralement une quantité relativement importante de sels d'acides gras qui peuvent avoir un caractère irritant pour des applications telles que le soin du visage. Par ailleurs, ces sticks laissent un film collant après application sur la peau.

Des gels rigides aqueux ont été décrits dans les documents WO-A-97/17055 et WO-A-97/17053. Toutefois, ces gels nécessitent l'emploi d'une concentration assez élevée de gélifiant ou font appel à une technique de préparation particulière, l'extrusion. En outre, les sticks décrits dans le document WO-A-97/17055 manquent de transparence et, du fait de la concentration élevée en gélifiant, manquent de fraîcheur et de douceur lors de l'application sur la peau, et ceux décrits dans le document WO-A-97/17053 doivent être hydratés au moment de l'emploi.

Dans le document EP-A-803245, il est décrit des compositions solides aqueuses contenant des polysaccharides thermoréversibles, un humectant et une phase poudreuse (charges). Toutefois, la présence d'une phase poudreuse peut entraîner les inconvénients suivants : présence d'une trace visible après application de la composition sur la peau et diminution du confort. En outre, quand on enlève la phase poudreuse de la composition décrite dans ce document, on obtient une composition qui n'a ni une solidité ni une stabilité suffisantes, et qui ne donne pas sur la peau un transfert satisfaisant (dépôt suffisant de produit).

Aussi, il subsiste le besoin d'une composition solide pour application topique qui ne présente pas les inconvénients de l'art antérieur.

Or, il se trouve que la demanderesse a découvert, de façon inattendue, un système gélifiant particulier à base d'hydrocolloïdes et de polymère associatif permettant de réaliser des compositions aqueuses rigides, homogènes et stables même à de faibles taux de gélifiant, et ne nécessitant pas obligatoirement la mise en oeuvre d'une technique de préparation particulière. Les compositions obtenues sont fraîches à l'application et permettent un bon dépôt de produit sur la peau tout en étant suffisamment solides. En outre, elles permettent l'application directe de produit sur la peau sans nécessiter de mouillage préalable.

Un polymère associatif est un polymère amphiphile comportant au moins une chaîne grasse, donc une partie hydrophobe, et au moins un motif hydrophile, donc une partie hydrophile.

La présente invention a ainsi pour objet une composition solide contenant dans une phase aqueuse, un système gélifiant comprenant (i) de la gomme de gellane, (ii) au moins un autre hydrocolloïde choisi dans le groupe formé par la gomme de xanthane, la carboxyméthylcellulose, l'hydroxypropylcellulose, la méthylcellulose, l'hydroxypropylméthylcellulose, l'hydroxyéthylcellulose, l'agar-agar, les carraghénanes, les alginates, la gomme de caroube, la gomme guar, la gomme arabique, la gomme karaya, la gomme adragante, la gomme de ghatti, les pectines, la gélatine, les caséinates, l'hydroxypropylguar, et (iii) au moins un polymère amphiphile comportant au moins une chaîne grasse et au moins un motif hydrophile.

Au sens de la présente invention, on entend par composition solide, toute composition présentant une résistance à la compression supérieure ou égale à 20 grammes, à température ambiante (20-25°C), après pénétration par une sonde cylindrique de révolution ayant un diamètre de 0,8 cm dans la matrice de la composition dans une épaisseur de 1 mm à une vitesse de 0,5 mm/s et retrait de ladite sonde de la matrice de la composition à une vitesse de 0,5 mm/s ; la résistance à la compression étant mesurée avec un analyseur du type "LFRA Texture Analyser" commercialisé par la Société STEVENS/MECHTRIC.

La composition solide de l'invention est apte à s'appliquer directement sur un support, c'est-à-dire qu'elle n'a pas besoin d'être mouillée pour s'appliquer sur le support et notamment sur la peau. On entend par "support" de la composition selon l'invention, toute surface sur laquelle on peut faire une application topique, notamment la peau, les fibres kératiniques telles que les cils et les cheveux, le cuir chevelu et les muqueuses telles que les lèvres.

Le système gélifiant utilisé selon l'invention permet, même en faible quantité, d'obtenir une composition solide ayant une résistance et une rigidité satisfaisantes tout en laissant un bon dépôt sur la peau.

En outre, contrairement aux compositions solides comportant une forte proportion de poudres, la composition de l'invention a l'avantage de ne pas laisser de dépôt poudreux visible lorsqu'elle est appliquée sur un support. Par ailleurs, elle présente la propriété d'être transparente ou translucide en l'absence d'huile.

De plus, la composition solide selon l'invention présente une bonne propriété de transfert, c'est-à-dire que lors d'une application sur un support, elle libère une quantité efficace de produit sur ce support, tout en étant particulièrement solide.

Dans la composition selon l'invention, la gomme de gellane est présente en une quantité d'au moins 1,5 % du poids total de la composition et par exemple en une quantité allant de 1,5 à 15 %, de préférence de 2 à 8 % et mieux de 2 à 4 % en poids par rapport au poids total de la composition.

Selon un mode préféré de réalisation de l'invention, l'hydrocolloïde (ii) est choisi de préférence parmi la gomme de xanthane, la gomme de caroube, la carboxyméthylcellulose et l'hydroxypropylguar et leurs mélanges.

Le ou les hydrocolloïdes (ii) sont présents dans la composition selon l'invention dans une quantité qui peut varier dans une large mesure. Ainsi, cette quantité peut aller par exemple de 0,1 à 10 %, de préférence de 1 à 5 % et mieux de 2 à 4 % en poids par rapport au poids total de la composition.

Selon les proportions et notamment lorsque la quantité totale d'hydrocolloïdes (gellane plus hydrocolloïde (ii)) dépasse 4 %, on peut de manière avantageuse réaliser le mélange dans un extrudeur à deux vis selon la technique décrite dans le document EP-A-667148.

Comme polymère amphiphile (iii), on peut utiliser dans la composition de l'invention tout polymère associatif. Dans ce polymère amphiphile, la partie hydrophobe peut être en nombre réduit vis-à-vis du reste de la chaîne polymérique, et peut se situer latéralement à la chaîne et être répartie de façon aléatoire (copolymères statistiques) ou répartie sous forme de séquences ou de greffons (copolymères blocs ou copolymères séquencés). Par ailleurs, on peut utiliser des polymères solubles dans l'eau ou hydrodispersibles ou encore des polymères "gonflables" dans l'eau.

Les polymères amphiphiles peuvent être de toute nature chimique ; on peut ainsi choisir des polymères d'origine naturelle, éventuellement modifiés ; des polymères radicalaires notamment vinyliques ou acryliques ; des polycondensats ; et leurs mélanges. Ils peuvent être ioniques ou non-ioniques, et sont de préférence anioniques ou non-ioniques.

Les polymères amphiphiles comportant au moins une chaîne grasse et au moins un motif hydrophile, utilisés selon l'invention sont choisis de préférence dans le groupe constitué par :
(1) les holosides modifiés par des groupements comportant au moins une chaîne grasse. Comme holosides modifiés, on peut citer à titre d'exemple :
   - les celluloses ou leurs dérivés, modifiés par des groupements comportant au moins une chaîne grasse, tels que des groupes alkyle, arylalkyle, alkylaryle ou leurs mélanges où les groupes alkyle ont de 8 à 22 atomes de carbone ;
   - les alkylhydroxyéthylcelluloses non-ioniques telles que les produits NATROSOL PLUS GRADE 330 CS®, POLYSURF 67® et ADX 401® (alkyle en C₁₆) vendus par la société AQUALON ;
   - les alkylhydroxyéthylcelluloses quaternisées (cationiques) telles que les produits QUATRISOFT LM 200®, QUATRISOFT LM-X 529-18-A®, QUATRISOFT LM-X 529-18-B® (alkyle en C₁₂) et QUATRISOFT LM-X 529-8® (alkyle en C₁₈) vendus par la société AMERCHOL et les produits CRODACEL QM®, CRODACEL QL® (alkyle en C₁₂) et CRODACEL QS® (alkyle en C₁₈) vendus par la société CRODA ;
   - les nonoxynylhydroxyéthylcelluloses non-ioniques tels que le produit AMERCELL HM-1500® vendu par la société AMERCHOL ;
   - les alkylcelluloses non-ioniques telles que le produit BERMOCOLL EHM 100® vendu par la société BEROL NOBEL ;
   - les polyalcools(C₁₂-C₁₈) saccharides tels que le produit EMULSAN® (mélange D-galactosamine/acide aminouronique) et le produit BIOSAN LPS-50® vendus par la société PETROFERM ;
   - les hydroxypropylguars modifiés par une chaîne grasse tel que le produit ESAFLOR HM 22® (modifié par une chaîne alkyle en C₂₂) vendu par la société LAMBERTI ; le produit MIRACARE XC 95-3® (modifié par une chaîne alkyle en C₁₄) et le produit RE 205-146 (modifié par une chaîne alkyle en C₂₀) vendus par la société RHONE-POULENC ;
(2) les copolymères d'anhydride maléique ou de l'un de ses dérivés et de monomères comportant au moins une chaîne grasse. On peut citer à titre d'exemple :
   - les copolymères N-octadécylvinyléther/anhydride maléique comme le produit GANTREZ AN-8194® vendu par la société ISP ;
   - les terpolymères acétate de vinyle/monomaléate d'isobutyle/néodécanoate de vinyle tels que les produits ACV-4033® et 9649-147® vendus par la société ISP, le produit MEYPRO-FIX 509® vendu par la société MEYHALL et les produits DENSODRIN BA et LIPODERM LIQUOR FP vendus par la société BASF ;
(3) les polyuréthannes et leurs dérivés comportant des groupements contenant au moins une chaîne grasse tels que par exemple les produits commerciaux suivants: RHEOLATE® 204, 205, 208, 210, 255 et 278 vendus par la société RHEOX; BERMODOL PUR 2130® vendu par la société BEROL NOBEL ; ACRYSOL SCT-275®, ACRYSOL RM-870®, ACRYSOL RM-825®, ACRYSOL 44® et ACRYSOL 46®, DW-1206 B®, DW-1206 F®, DW-1206 G® et DW-1206 J® vendus par la société ROHM & HAAS ; DAPRAL T 212® vendu par la société AKZO ; SER-AD FX 1100® vendu par la société HULS ; BORCHIGEL LW.44® et BORCHIGEL L.75.N® vendus par la société BORCHERS ;
(4) les copolymères de l'acide crotonique et de monomères comportant au moins une chaîne grasse, tels que les terpolymères acétate de vinyle/acide crotonique/stéarate d'allyle ;
(5) les copolymères de N-vinylpyrrolidone et de monomères comportant au moins une chaîne grasse, tels que des oléfines substituées par un radical alkyle comportant une longue chaîne hydrocarbonée comme par exemple les produits ANTARON V216® et ANTARON V220® vendus par la société ISP ;
(6) les copolymères d'acide (méth)acrylique et de monomères comportant au moins une chaîne grasse ; ces monomères sont choisis parmi les monomères hydrophobes à chaîne grasse, les monomères amphiphiles comportant une partie hydrophobe à chaîne grasse et une partie hydrophile ou bien leurs mélanges. Comme copolymères de ce type, on peut citer à titre d'exemples :
   - les copolymères réticulés d'acide acrylique/acrylate d'alkyle en C₁₀-C₃₀ tels que les produits PEMULEN TR 1®, PEMULEN TR 2®, CARBOPOL 1382®, CARBOPOL 1342® et CARBOPOL ETD 2020® vendus par la société GOODRICH ;
   - les copolymères acide (méth)acrylique/acrylate d'éthyle/acrylate d'alkyle tels que le produit ACUSOL 823® vendu par la société ROHM & HAAS et le produit IMPERON R® vendu par la société HOECHST ;
   - les copolymères réticulés acide acrylique/isodécanoate de vinyle tels que le produit STABYLEN 30® vendu par la société 3V ;
   - les terpolymères acide acrylique/vinylpyrrolidone/méthacrylate de lauryle tels que les produits ACRYLIDONE LM®, ACP-1184®, ACP-1194® vendus par la société ISP ;
   - les copolymères acide acrylique/(méth)acrylate de lauryle tels que les produits COATEX SX® vendus par la société COATEX ;
   - les terpolymères acide (méth)acrylique/acrylate d'alkyle/alkyl polyéthoxylé allyl éther tels que les produits RHEOVIS® -CR, -CR₃, -CR₂ et -CRX vendus par la société ALLIED COLLOIDS ;
   - les terpolymères acide méthacrylique/acrylate d'éthyle/stéaryl polyéthoxylé allyl éther tels que les produits SALCARE-SC90® et -SC80® vendus par la société ALLIED COLLOIDS ;
   - les terpolymères acide méthacrylique/acrylate d'éthyle/acrylate de lauryle polyoxyéthyléné tels que le produit RHEO 2000® vendu par la société COATEX ;
   - les terpolymères acide méthacryliquelacrylate d'éthyle/méthacrylate de stéaryle polyoxyéthyléné tels que les produits ACRYSOL 22®, ACRYSOL 25® et DW-1206A® vendus par la société ROHM & HAAS ;
   - les copolymères acide méthacrylique/acrylate d'éthyle/acrylate de nonylphénol polyoxyéthyléné tels que le produit RHEO 3000® vendu par la société COATEX ;
   - les copolymères acide acrylique/monoitaconate de stéaryle polyoxyéthyléné ou les copolymères acide acrylique/monoitaconate de cétyle polyoxyéthyléné, tels que les produits 8069-72A et 8069-72B vendus par la société NATIONAL STARCH ;
   - les copolymères acide méthacrylique/acrylate de butyle/monomère hydrophobe comportant au moins une chaîne grasse, tels que le produit 8069-146A vendu par la société NATIONAL STARCH ;
   - les terpolymères acide acrylique/acrylate d'alkyle en C₁₅/acrylate de polyéthylèneglycol (28 moles d'oxyde d'éthylène), tels que le produit DAPRAL GE 202® vendu par la société AKZO ;
   - les sels d'un ester d'acide gras partiel d'un copolymère acide acrylique/diméthyléthanolamine, tels que le produit DAPRAL GE 202 DMA® vendu par la société AKZO ;
   - les copolymères acide acrylique/acrylate/monomère amphiphile comportant une chaîne grasse à groupements uréthane, tels que le produit ADDITOL VXW 1312® vendu par la société HOECHST ;
   - les copolymères acryliques modifiés par des groupes hydrophobes à chaîne grasse, tels que le produit ACUSOL 102® vendu par la société ROHM & HAAS ;
(7) les copolymères non-ioniques de (méth)acrylate d'alkyle inférieur (C₁-C₆) et de monomères amphiphiles comportant une chaîne grasse comme par exemple les copolymères de méthacrylate de méthyle/acrylate de stéaryle polyoxyéthyléné tels que le produit ANTIL 208® vendu par la société GOLDSCHMIDT ;
(8) les copolymères non-ioniques de (méth)acrylates hydrophiles et de monomères hydrophobes à chaîne grasse comme par exemple les copolymères méthacrylate de polyéthylèneglycol/méthacrylate de méthyle.

Selon un mode préféré de réalisation de l'invention, on choisit plus particulièrement le polymère amphiphile parmi les holosides modifiés et les polyuréthannes.

On peut utiliser dans la composition de l'invention soit un polymère amphiphile soit un mélange de polymères amphiphiles.

Les polymères amphiphiles sont utilisés dans la composition selon l'invention en une quantité efficace pour obtenir le résultat escompté. De préférence, la quantité de polymère(s) amphiphile(s) va de 0,05 à 20 % en poids par rapport au poids total de la composition. Plus préférentiellement, cette quantité varie de 0,1 à 10 % en poids et encore plus préférentiellement de 0,2 à 5 % en poids par rapport au poids total de la composition.

Le système gélifiant comprenant les composés (i), (ii) et (iii) dans la composition de l'invention est utilisé en une quantité efficace pour obtenir le résultat escompté. Il représente de préférence de 2 à 30 % et mieux de 2 à 10 % en poids par rapport au poids total de la composition.

La phase aqueuse de la composition selon l'invention représente généralement de 60 à 97 %, et de préférence de 80 à 95 % en poids par rapport au poids total de la composition.

Il est possible de modifier la rigidité des compositions selon l'invention en y ajoutant un ou plusieurs sels qui vont augmenter cette rigidité. Ces sels peuvent être choisis parmi les sels des métaux mono-, di- ou trivalents, et plus particulièrement les sels de métal alcalin et alcalino-terreux et en particulier les sels de sodium et de calcium. Les ions constituant ces sels peuvent être choisis par exemple parmi les carbonates, les bicarbonates, les sulfates, les glycérophosphates, les borates, les chlorures, les nitrates, les acétates, les hydroxydes, les persulfates ainsi que les sels d'a-hydroxyacides (citrates, tartrates, lactates, malates) ou d'acides de fruits, ou encore les sels d'acides aminés (aspartate, arginate, glycocholate, fumarate).

De préférence, le sel est choisi parmi le chlorure de sodium, de calcium, de magnésium, de strontium, de néodyme ou de manganèse, le nitrate de calcium, de magnésium ou de strontium, le borate de calcium ou de magnésium, le sulfate de magnésium ou de calcium, l'acétate de calcium ou de magnésium, et leurs mélanges.

La quantité de sel(s) peut aller de 0,01 à 5 % et de préférence de 0,1 à 2 % du poids total de la composition.

Selon un mode préféré de réalisation de l'invention, la composition comprend en outre au moins un solvant autre que l'eau. Comme solvants, on peut citer les alcools primaires comportant de 1 à 4 atomes de carbone, tels que l'éthanol et l'isopropanol, les glycols tels que le propylène glycol, le butylène glycol, le dipropylène glycol, le diéthylène glycol, les éthers de glycol tels que les alkyl(C₁-C₄)éther de mono, di- ou tripropylène glycol, mono, di- ou triéthylène glycol, et leurs mélanges. La quantité de solvant(s) peut aller de 0,01 à 20 % et de préférence de 0,1 à 5 % en poids par rapport au poids total de la composition.

La composition solide selon l'invention est une composition destinée à une application topique, notamment cosmétique ou dermatologique. Une telle composition comporte un milieu physiologiquement acceptable, en particulier pour la peau, y compris le cuir chevelu, les muqueuses, les ongles et/ou les fibres kératiniques (cheveux ou cils).

Selon un mode particulier de réalisation de l'invention, la composition comprend en outre au moins une huile, cette addition d'huile apportant un plus grand confort lors de l'application de la composition sur la peau.

Parmi les huiles utilisables, on peut citer les huiles minérales, les huiles d'origine végétale, les huiles d'origine animale, les huiles de synthèse telles que les esters gras, les huiles de silicone telles que les huiles de silicone volatile, les polyméthylsiloxanes, les polyméthylphénylsiloxanes, les polysiloxanes modifiés par des acides gras, des alcools gras ou des polyoxyalkylènes, les silicones fluorées et les huiles perfluorées. On peut ajouter d'autres corps gras tels que les acides gras, les alcools gras et les cires.

La ou les huiles et les autres corps gras éventuellement présents constituent la phase grasse.

La phase grasse peut être présente dans des proportions allant, par exemple, jusqu'à 30 %, de préférence de 0,1 à 20 % et mieux de 0,5 à 10 % en poids par rapport au poids total de la composition, ces proportions variant selon l'application choisie.

La phase grasse peut être introduite dans la phase aqueuse en présence d'un ou de plusieurs tensioactifs pour assurer une meilleure dispersion.

Les compositions selon l'invention peuvent donc également contenir un ou plusieurs tensioactifs non ioniques, anioniques, cationiques ou amphotères, habituellement utilisés dans les domaines cosmétique et/ou dermatologique. Les quantités en agent(s) tensioactif(s) peuvent aller de 0,05 à 8 % et mieux de 0,05 à 5 % en poids par rapport au poids total de la composition.

Les compositions selon l'invention peuvent aussi contenir des additifs habituellement utilisés dans les domaines cosmétique et/ou dermatologique. On peut en particulier citer les agents antioxydants ou anti-radicaux libres, les colorants hydrosolubles tels que FD&C Red n°4 et D&C Green n°5, ou encore les colorants liposolubles si la composition comporte une phase grasse, les actifs hydrophiles ou lipophiles, les parfums, les charges.

Les actifs peuvent être choisis par exemple parmi les agents hydratants ou humectants tels que les polyols et notamment la glycérine, les filtres UV, les agents antipelliculaires, les agents conditionneurs, les actifs déodorants, les dépigmentants ou les agents blanchissants, les agents tenseurs et antirides, les latex et les pseudolatex, et tout autre actif approprié à la finalité du produit solide considéré.

Comme latex et pseudolatex, on peut citer par exemple les dispersions de polymères synthétiques de type polycondensat ou de type radicalaire. Comme polymères constituant le latex ou le pseudolatex, on peut citer les polyuréthannes anioniques, cationiques, non ioniques ou amphotères, les polyuréthannes-acryliques, les polyuréthannes-polyvinylpyrrolidones, les polyester-polyuréthannes, les polyéther-polyuréthannes, les polyurées, les polymères acryliques, les copolymères acryliques, les polymères d'acide isophtalique sulfoné, ainsi que les polymères résultant de la polymérisation radicalaire d'un ou plusieurs monomères radicalaires. Comme polymère synthétique approprié pour être utilisé comme latex, on peut citer notamment les dispersions de polyester-polyuréthanne et de polyéther-polyuréthanne, commercialisées sous les dénominations "Sancure 2060®" (polyester-polyuréthanne), "Sancure 2255®" (polyester-polyuréthanne), "Sancure 815®" (polyester-polyuréthanne), "Sancure 878®" (polyéther-polyuréthanne) et "Sancure 861®" (polyéther-polyuréthanne) par la société SANNCOR, sous les dénominations "Neorez R974®" (polyester-polyuréthanne), "Neorez R981®" (polyester-polyuréthanne), "Neorez R970®" (polyéther-polyuréthanne) par la société ICI, et la dispersion de copolymère acrylique, commercialisée sous la dénomination "Neocryl XK-90®" par la société ZENECA.

Comme charges, on peut citer par exemple les poudres, les pigments et les colorants insolubles, et en particulier les poudres de talc, d'amidon, de polymères et copolymères d'acrylates, de mica, de kaolin, de polyamide (nylon), de polyéthylène, de silice et de silicone.

Ces additifs peuvent être présents dans la composition finale en une quantité allant de 0 à 30 %, de préférence de 0,5 à 20 % et encore plus particulièrement de 0,5 à 10 % en poids par rapport au poids total de la composition.

Bien entendu, l'homme de l'art veillera à choisir ce ou ces éventuels additifs et/ou leurs quantités de manière telle que les propriétés avantageuses attachées intrinsèquement à la composition conforme à l'invention ne soient pas, ou substantiellement pas, altérées par la ou les adjonctions envisagées.

Les compositions selon l'invention peuvent constituer des produits pour le soin et/ou le conditionnement et/ou l'hygiène de la peau, des muqueuses, du cuir chevelu et/ou des cheveux. Parmi les produits de soin, de conditionnement ou d'hygiène en forme de pain, de stick ou de crayon, on peut mentionner, par exemple, en capillaire, des gels solides de coiffage et/ou de mise en forme des cheveux ; en soin de la peau, des produits hydratants, des produits amincissants, des produits dépigmentants et blanchissants, des produits pour le soin des lèvres ; pour l'hygiène du visage et/ou du corps, des produits de rasage, des déodorants.

Un autre objet de l'invention réside dans un procédé de traitement cosmétique pour le soin et/ou le conditionnement et/ou l'hygiène de la peau, des cheveux, du cuir chevelu et/ou des muqueuses, consistant à appliquer sur la peau, les cheveux, le cuir chevelu et/ou les muqueuses une composition solide telle que définie ci-dessus.

Les compositions selon l'invention peuvent constituer aussi des produits de maquillage, tels que des rouges à lèvres, des fonds de teints, des ombres à paupières, des fards à joues, des anti-cernes, des mascaras, des crayons du contour des lèvres, des crayons du contour des yeux, des sticks pour la teinture de mèches de cheveux. Elles peuvent notamment constituer des produits de maquillage "sans transfert", c'est-à-dire qui déposent un film qui, après application, ne transfère pas ou ne migre pas ou ne tache pas une surface avec laquelle le produit de maquillage appliqué sur la peau peut être par la suite mis en contact (vêtement, verre, tasse, etc...).

Aussi la présente invention a également pour objet l'utilisation de la composition selon l'invention pour l'obtention d'un produit de maquillage sans transfert. Quand le produit de maquillage contient en outre un latex ou un pseudolatex, on obtient un produit présentant une bonne rémanence.

La présente invention a encore pour objet un procédé de maquillage de la peau et/ou des fibres kératiniques, consistant à appliquer sur le visage, les lèvres, le contour des yeux, les joues, le contour des lèvres, les cils, les sourcils, les cheveux et/ou les paupières, une composition solide telle que définie ci-dessus.

Les compositions de l'invention peuvent aussi constituer des patchs destinés à être appliqués directement sur la peau. Ces patchs peuvent comprendre la composition selon l'invention seule ou incorporée dans une structure composite sous forme de couches, pouvant comprendre notamment, outre la composition de l'invention, une couche support et/ou une couche détachable de protection.

Aussi, l'invention a également pour objet un patch, caractérisé en ce qu'il comprend une composition telle que définie ci-dessus.

Les exemples qui suivent servent à illustrer l'invention. Les pourcentages sont exprimés en poids, sauf mention contraire.

### Exemple 1 : Stick hydratant

- Gomme de gellane 2 %
- Gomme de xanthane 1 %
- Chlorure de sodium 1 %
- Ethanol 1 %
- Polyuréthanne (SER-AD FX 1100®) 0,5 %
- Eau qsp 100 %

Le stick est préparé par mélange des constituants à 80°C sous agitation et coulage à chaud.

Le stick obtenu est transparent et dépose un film frais lors de l'application sur la peau. En outre, il a une bonne dureté (700 g/cm²) et laisse un bon dépôt sur la peau.

**Exemple comparatif** : la même composition sans polyuréthanne a une dureté satisfaisante (800 g/cm²), mais ne laisse pratiquement pas de dépôt sur la peau.

## Revendications

1. Composition solide cosmétique ou dermatologique contenant dans une phase aqueuse, un système gélifiant comprenant (i) de la gomme de gellane, (ii) au moins un autre hydrocolloïde choisi dans le groupe formé par la gomme de xanthane, la carboxyméthylcellulose, l'hydroxypropylcellulose, la méthylcellulose, l'hydroxypropylméthylcellulose, l'hydroxyéthylcellulose, l'agar-agar, les carraghénanes, les alginates, la gomme de caroube, la gomme guar, la gomme arabique, la gomme karaya, la gomme adragante, la gomme de ghatti, les pectines, la gélatine, les caséinates, l'hydroxypropylguar, et (iii) au moins un polymère amphiphile comportant au moins une chaine grasse et au moins un motif hydrophile.

2. Composition selon la revendication 1, **caractérisée en ce que** l'hydrocolloïde (ii) est choisi dans le groupe formé par la gomme de xanthane, la gomme de caroube, la carboxyméthylcellulose, l'hydroxypropylguar et leurs mélanges.

3. Composition selon l'une quelconque des revendications précédentes, **caractérisée en ce que** la gomme de gellane est présente en une quantité allant de 1,5 à 15 % et de préférence de 2 à 8 % en poids par rapport au poids total de la composition.

4. Composition selon l'une quelconque des revendications précédentes, **caractérisée en ce que** la quantité d'hydrocolloïde (ii) va de 0,1 à 10 % et de préférence de 1 à 5 % en poids par rapport au poids total de la composition.

5. Composition selon l'une quelconque des revendications précédentes, **caractérisée en ce que** le polymère amphiphile comportant au moins une chaîne grasse et au moins un motif hydrophile est choisi parmi :
(1) les holosides modifiés par des groupes comportant au moins une chaîne grasse ;
(2) les copolymères d'anhydride maléique ou de l'un de ses dérivés et de monomères comportant au moins une chaîne grasse ;
(3) les polyuréthannes et leurs dérivés comportant des groupements comportant au moins une chaîne grasse ;
(4) les copolymères de l'acide crotonique et de monomères comportant au moins une chaîne grasse ;
(5) les copolymères de N-vinylpyrrolidone et de monomères comportant au moins une chaîne grasse ;
(6) les copolymères d'acide (méth)acrylique et de monomères comportant au moins une chaîne grasse ;
(7) les copolymères non-ioniques de (méth)acrylate d'alkyle inférieur (C₁-C₆) et de monomères amphiphiles comportant une chaîne grasse ;
(8) les copolymères non-ioniques de (méth)acrylates hydrophiles et de monomères hydrophobes à chaîne grasse.

6. Composition selon l'une quelconque des revendications précédentes, **caractérisée en ce que** la quantité de polymère amphiphile va de 0,05 à 20 % et de préférence de 0,1 à 10 % en poids par rapport au poids total de la composition.

7. Composition selon l'une quelconque des revendications précédentes **caractérisée en ce que** le système gélifiant comprenant (i), (ii) et (iii) représente de 2 à 30 % et de préférence de 2 à 10 % en poids par rapport au poids total de la composition.

8. Composition selon l'une quelconque des revendications précédentes, **caractérisée en ce que** la phase aqueuse représente de 60 à 97 % en poids par rapport au poids total de la composition.

9. Composition selon l'une quelconque des revendications précédentes, **caractérisée en ce qu'**elle contient en outre au moins un sel et/ou un solvant autre que l'eau.

10. Composition selon la revendication précédente, **caractérisée en ce que** la quantité de sel(s) va de 0,01 à 5 % en poids par rapport au poids total de la composition.

11. Composition selon la revendication 9, **caractérisée en ce que** la quantité de solvant(s) va de 0,01 à 20 % en poids par rapport au poids total de la composition.

12. Composition selon l'une quelconque des revendications précédentes, **caractérisée en ce qu'**elle comprend en outre une phase grasse.

13. Composition selon la revendication précédente, **caractérisée en ce que** la phase grasse est présente en une quantité allant jusqu'à 30 % et de préférence de 0,1 à 20% en poids par rapport au poids total de la composition.

14. Composition selon l'une quelconque des revendications précédentes, **caractérisée en ce qu'**elle comprend, en outre, au moins un additif choisi dans le groupe formé par les agents antioxydants, les agents anti-radicaux libres, les colorants hydrosolubles ou liposolubles, les charges, les actifs lipophiles ou hydrophiles, les parfums.

15. Composition selon la revendication précédente, **caractérisée en ce que** l'actif est choisi parmi les hydratants, les filtres UV, les agents antipelliculaires, les agents conditionneurs, les actifs déodorants, les dépigmentants, les agents blanchissants, les agents tenseurs et antirides, les latex, les pseudolatex.

16. Produit de maquillage, **caractérisé en ce qu'**il comprend une composition selon l'une quelconque des revendications précédentes.

17. Produit pour le soin et/ou le conditionnement et/ou l'hygiène de la peau, des muqueuses, du cuir chevelu ou des cheveux, **caractérisé en ce qu'**il comprend une composition solide selon l'une quelconque des revendications 1 à 15.

18. Patch, **caractérisé en ce qu'**il comprend une composition selon l'une quelconque des revendications 1 à 15.

19. Procédé de maquillage de la peau et/ou des fibres kératiniques, consistant à appliquer sur le visage, les lèvres, le contour des yeux, les joues, le contour des lèvres, les cils, les sourcils, les cheveux et/ou les paupières, une composition solide selon l'une quelconque des revendications 1 à 15.

20. Procédé de traitement non-thérapeutique pour le soin et/ou le conditionnement et/ou l'hygiène de la peau, des cheveux, du cuir chevelu et/ou des muqueuses, consistant à appliquer sur la peau, les cheveux, le cuir chevelu et/ou les muqueuses une composition solide non-thérapeutique selon l'une quelconque des revendications 1 à 15.

21. Utilisation non-thérapeutique d'une composition non-thérapeutique selon l'une quelconque des revendications 1 à 15 pour obtenir un pr uit de maquillage sans transfert.

22. Utilisation d'une composition selon l'une quelconque des revendications 1 à 15, pour la fabrication d'une composition dermatologique destinée au soin de la peau, des cheveux, du cuir chevelu et/ou des muqueuses.

## Patentansprüche

1. Feste kosmetische oder dermatologische Zusammensetzung, die in einer wäßrigen Phase ein Gelbildnersystem enthält, das (i) Gellangummi, (ii) mindestens ein weiteres Hydrokolloid, das unter Xanthangummi, Carboxymethylcellulose, Hydroxypropylcellulose, Methylcellulose, Hydroxypropylmethylcellulose, Hydroxyethylcellulose, Agar-Agar, Carrageenanen, Alginaten, Johannisbrotbaum-Kernmehl, Guar-Gummi, Gummi arabicum, Karaya-Gummi, Tragant, Ghatti gummi, Pektinen, Gelatine, Caseinaten und Hydroxypropylguar ausgewählt ist, und (iii) mindestens ein amphiphiles Polymer enthält, das mindestens eine Fettkette und mindestens eine hydrophile Gruppe aufweist.

2. Zusammensetzung nach Anspruch 1, **dadurch gekennzeichnet, daß** das Hydrokolloid (ii) unter Xanthangummi, Johannisbrotbaum-Kernmehl, Carboxymethylcellulose, Hydroxypropylguar und deren Gemischen ausgewählt ist.

3. Zusammensetzung nach einem der vorhergehenden Ansprüche, **dadurch gekennzeichnet, daß** das Gellangummi in einem Mengenanteil von 1,5 bis 15 Gew.-% und vorzugsweise 2 bis 8 Gew.-%, bezogen auf das Gesamtgewicht der Zusammensetzung, vorliegt.

4. Zusammensetzung nach einem der vorhergehenden Ansprüche, **dadurch gekennzeichnet, daß** der Mengenanteil des Hydrokolloids (ii) im Bereich von 0,1 bis 10 Gew.-% und vorzugsweise 1 bis 5 Gew.-%, bezogen auf das Gesamtgewicht der Zusammensetzung, liegt.

5. Zusammensetzung nach einem der vorhergehenden Ansprüche, **dadurch gekennzeichnet, daß** das amphiphile Polymer, das mindestens eine Fettkette und mindestens eine hydrophile Gruppe aufweist, ausgewählt ist unter:
(1) Holosiden, die mit Gruppen modifiziert sind, die mindestens eine Fettkette aufweisen;
(2) den Copolymeren von Maleinsäureanhydrid oder einem seiner Derivate und Monomeren, die mindestens eine Fettkette aufweisen;
(3) Polyurethanen und deren Derivaten, die Gruppen tragen, die mindestens eine Fettkette aufweisen;
(4) Copolymeren von Crotonsäure und Monomeren, die mindestens eine Fettkette aufweisen;
(5) Copolymeren von N-Vinylpyrrolidon und Monomeren, die mindestens eine Fettkette aufweisen;
(6) Copolymeren von (Meth)acrylsäure und Monomeren, die mindestens eine Fettkette aufweisen;
(7) nichtionischen Copolymeren von Alkyl(meth)acrylaten (niedere C₁₋₆-Alkylguppe) und amphiphilen Monomeren mit Fettkette;
(8) nichtionischen Copolymeren von hydrophilen (Meth)acrylaten und hydrophoben Monomeren mit Fettkette.

6. Zusammensetzung nach einem der vorhergehenden Ansprüche, **dadurch gekennzeichnet, daß** der Mengenanteil des amphiphilen Polymers im Bereich von 0,05 bis 20 % und vorzugsweise 0,1 bis 10 Gew.-%, bezogen auf das Gesamtgewicht der Zusammensetzung, liegt.

7. Zusammensetzung nach einem der vorhergehenden Ansprüche, **dadurch gekennzeichnet, daß** das Gelbildnersystem, das (i), (ii) und (iii) enthält, 2 bis 30 % und vorzugsweise 2 bis 10 Gew.-%, bezogen auf das Gesamtgewicht der Zusammensetzung, ausmacht.

8. Zusammensetzung nach einem der vorhergehenden Ansprüche, **dadurch gekennzeichnet, daß** die wäßrige Phase 60 bis 97 Gew.-%, bezogen auf das Gesamtgewicht der Zusammensetzung, ausmacht.

9. Zusammensetzung nach einem der vorhergehenden Ansprüche, **dadurch gekennzeichnet, daß** sie ferner mindestens ein Salz und/oder ein Lösungsmittel, das von Wasser verschieden ist, enthält.

10. Zusammensetzung nach dem vorhergehenden Anspruch, **dadurch gekennzeichnet, daß** der Mengenanteil des Salzes oder der Salze im Bereich von 0,01 bis 5 Gew.-%, bezogen auf das Gesamtgewicht der Zusammensetzung, liegt.

11. Zusammensetzung nach Anspruch 9, **dadurch gekennzeichnet, daß** der Mengenanteil des oder der Lösungsmittel im Bereich von 0,01 bis 20 Gew.-%, bezogen auf das Gesamtgewicht der Zusammensetzung, liegt.

12. Zusammensetzung nach einem der vorhergehenden Ansprüche, **dadurch gekennzeichnet, daß** sie ferner mindestens eine Fettphase aufweist.

13. Zusammensetzung nach dem vorhergehenden Anspruch, **dadurch gekennzeichnet, daß** die Fettphase in einem Mengenanteil von bis zu 30 % und vorzugsweise 0,1 bis 20 Gew.-%, bezogen auf das Gesamtgewicht der Zusammensetzung, vorliegt.

14. Zusammensetzung nach einem der vorhergehenden Ansprüche, **dadurch gekennzeichnet, daß** sie außerdem mindestens einen Zusatzstoff enthält, der unter den Antioxidantien, Mitteln gegen freie Radikale, wasserlöslichen oder fettlöslichen Färbemitteln, Füllstoffen, lipophilen oder hydrophilen Wirkstoffen und Parfums ausgewählt ist.

15. Zusammensetzung nach dem vorhergehenden Anspruch, **dadurch gekennzeichnet, daß** der Wirkstoff unter den Hydratisierungsmitteln, UV-Filtern, Mitteln gegen Schuppen, Konditioniermitteln, Deodorants, depigmentierenden Mitteln, bleichenden Mitteln, straffenden Mitteln, Antifaltenmitteln, Latices und Pseudolatices ausgewählt ist.

16. Produkt zum Schminken, **dadurch gekennzeichnet, daß** es eine Zusammensetzung nach einem der vorhergehenden Ansprüche enthält.

17. Produkt zur Pflege und/oder Konditionierung und/oder Hygiene der Haut, der Schleimhäute, der Kopfhaut oder der Haare, **dadurch gekennzeichnet, daß** es eine feste Zusammensetzung nach einem der Ansprüche 1 bis 15 enthält.

18. Pflaster, **dadurch gekennzeichnet, daß** es eine Zusammensetzung nach einem der Ansprüche 1 bis 15 enthält.

19. Verfahren zum Schminken der Haut und/oder der Keratinfasern, das darin besteht, auf das Gesicht, die Lippen, die Augenkonturen, die Wangen, die Lippenkonturen, die Wimpern, die Augenbrauen, die Haare und/oder die Lider eine feste Zusammensetzung nach einem der Ansprüche 1 bis 15 aufzubringen.

20. Verfahren zur nicht therapeutischen Behandlung, zur Pflege und/oder Konditionierung und/oder Hygiene der Haut, der Haare, der Kopfhaut und/oder der Schleimhäute, das darin besteht, auf die Haut, die Haare, die Kopfhaut und/oder die Schleimhäute eine feste nicht therapeutische Zusammensetzung nach einem der Ansprüche 1 bis 15 aufzutragen.

21. Nicht therapeutische Verwendung einer nicht therapeutischen Zusammensetzung nach einem der Ansprüche 1 bis 15 zur Herstellung eines Produkts zum Schminken ohne Transfer.

22. Verwendung einer Zusammensetzung nach einem der Ansprüche 1 bis 15 zur Herstellung einer dermatologischen Zusammensetzung, die zur Pflege der Haut, der Haare, der Kopfhaut und/oder der Schleimhäute vorgesehen ist.

## Claims

1. Cosmetic or dermatological solid composition containing, in an aqueous phase, a gelling system comprising (i) gellan gum, (ii) at least one other hydrocolloid chosen from the group formed by xanthan gum, carboxymethylcellulose, hydroxypropylcellulose, methylcellulose, hydroxypropylmethylcellulose, hydroxyethylcellulose, agar-agar, carrageenans, alginates, carob gum, guar gum, gum arabic, karaya gum, gum tragacanth, ghatti gum, pectins, gelatin, caseinates and hydroxypropylguar, and (iii) at least one amphiphilic polymer comprising at least one fatty chain and at least one hydrophilic unit.

2. Composition according to Claim 1, **characterized in that** the hydrocolloid (ii) is chosen from the group formed by xanthan gum, carob gum, carboxymethylcellulose and hydroxypropylguar, and mixtures thereof.

3. Composition according to either of the preceding claims, **characterized in that** the gellan gum is present in an amount ranging from 1.5 to 15% and preferably from 2 to 8% by weight relative to the total weight of the composition.

4. Composition according to any one of the preceding claims, **characterized in that** the amount of hydrocolloid (ii) ranges from 0.1 to 10% and preferably from 1 to 5% by weight relative to the total weight of the composition.

5. Composition according to any one of the preceding claims, **characterized in that** the amphiphilic polymer comprising at least one fatty chain and at least one hydrophilic unit is chosen from:
(1) holosides modified with groups comprising at least one fatty chain;
(2) copolymers of maleic anhydride or of a derivative thereof and of monomers comprising at least one fatty chain;
(3) polyurethanes and derivatives thereof comprising groups comprising at least one fatty chain;
(4) copolymers of crotonic acid and of monomers comprising at least one fatty chain;
(5) copolymers of N-vinylpyrrolidone and of monomers comprising at least one fatty chain;
(6) copolymers of (meth)acrylic acid and of monomers comprising at least one fatty chain;
(7) nonionic copolymers of lower (C₁-C₆)alkyl (meth)acrylate and of amphiphilic monomers comprising a fatty chain;
(8) nonionic copolymers of hydrophilic (meth)acrylates and of hydrophobic monomers containing a fatty chain.

6. Composition according to any one of the preceding claims, **characterized in that** the amount of amphiphilic polymer ranges from 0.05 to 20% and preferably from 0.1 to 10% by weight relative to the total weight of the composition.

7. Composition according to any one of the preceding claims, **characterized in that** the gelling system comprising (i), (ii) and (iii) represents from 2 to 30% and preferably from 2 to 10% by weight relative to the total weight of the composition.

8. Composition according to any one of the preceding claims, **characterized in that** the aqueous phase represents from 60 to 97% by weight relative to the total weight of the composition.

9. Composition according to any one of the preceding claims, **characterized in that** it also contains at least one salt and/or a solvent other than water.

10. Composition according to the preceding claim, **characterized in that** the amount of salt(s) ranges from 0.01 to 5% by weight relative to the total weight of the composition.

11. Composition according to Claim 9, **characterized in that** the amount of solvent(s) ranges from 0.01 to 20% by weight relative to the total weight of the composition.

12. Composition according to any one of the preceding claims, **characterized in that** it also comprises a fatty phase.

13. Composition according to the preceding claim, **characterized in that** the fatty phase is present in an amount ranging up to 30% and preferably from 0.1 to 20% by weight relative to the total weight of the composition.

14. Composition according to any one of the preceding claims, **characterized in that** it also comprises at least one additive chosen from the group formed from antioxidants, free-radical scavengers, water-soluble or liposoluble dyes, fillers, lipophilic or hydrophilic active agents and fragrances.

15. Composition according to the preceding claim, **characterized in that** the active agent is chosen from hydrating agents, UV screening agents, antidandruff agents, conditioners, deodorant active agents, depigmenting agents, bleaching agents, tensioning agents and anti-wrinkle agents, latices and pseudolatices.

16. Make-up product, **characterized in that** it comprises a composition according to any one of the preceding claims.

17. Product for the care and/or conditioning and/or hygiene of the skin, mucous membranes, the scalp or the hair, **characterized in that** it comprises a solid composition according to any one of Claims 1 to 15.

18. Patch, **characterized in that** it comprises a composition according to any one of Claims 1 to 15.

19. Process for making up the skin and/or keratin fibres, which consists in applying a solid composition according to any one of Claims 1 to 15 to the face, the lips, the contour of the eyes, the cheeks, the contour of the lips, the eyelashes, the eyebrows, the hair and/or the eyelids.

20. Non-therapeutic treatment process for the care and/or conditioning and/or hygiene of the skin, the hair, the scalp and/or mucous membranes, this process consisting in applying a non-therapeutic solid composition according to any one of Claims 1 to 15 to the skin, the hair, the scalp and/or mucous membranes.

21. Non-therapeutic use of a non-therapeutic composition according to any one of Claims 1 to 15 to obtain a transfer-free make-up product.

22. Use of a composition according to any one of claims 1 to 15, for the manufacture of a dermatological composition to care for the skin, the hair, the scalp and/or mucous membranes.
